# EUROPEAN PATENT APPLICATION

(11) **EP 3 205 259 A1**
(43) Date of publication of application: **16.08.2017**
(21) Application number: 16382061.6
(22) Date of filing: 15.02.2016
(51) Int. Cl.: A61B 3/06, G06F 3/01, G09G 3/34, A61B 3/10

(54) **DETECTOR AND METHOD FOR DETECTING THE LEVEL OF AVERSION OF THE OCULAR SYSTEM TO LIGHT SOURCE**

(71) Applicant: Universidad Complutense De Madrid, 28040 Madrid (ES); Tecnología Sostenible y Responsable SL, 28850 Torrejon de Ardoz (ES)
(72) Inventor: Sánchez Ramos, Celia, 28040 Madrid (ES); García Manchado, Nilo, 28850 Torrejón de Ardoz (ES)
(74) Representative: Sahuquillo Huerta, Jesús

(57) **Abstract**

Method and electronic device for detecting the level of light emission that a light source emits into a user's ocular system; measuring the user's level of aversion to the amount of light received in his/her ocular system through analysis of his/her eye reaction; and comparing the level of aversion to the amount of light received by his/her ocular system measured in step (b) to the level of aversion acceptable for that user and stored in a memory (102) of an electronic device (100).

## Description

The object of the present invention is a detector and a method of detecting the level of aversion (i.e. the level from which is uncomfortable) to a light source by a human eye.

### State of the art

Defining a level of aversion to a light source is crucial for the proper functioning of the ocular system. By "light source" in the present invention means any device or system capable of emitting an intense light in the visible spectrum and, especially, means any type of screen with own lighting from any computer, electronic, or similar system.

The screens are part of our lives today and its light has become a predominant source for the human ocular system. Currently, according to various estimates, there are more LED-technology screens than people in the world. In addition, this is combined with our time of exposure to that light exceed an average of eight hours in most of the countries. The problem is increasing with the youngest, since they are usually surrounded by screens and their ocular system is still under development.

The new LED screens can emit up to five times more high-energy light. This light is related to three types of risk, those relating to melatonin, those retinal and the set of symptoms called CVS (*Computer Vision Syndrome*) as headaches, red eyes, among others.

The mechanisms that the screens have to vary the amount of light emitted vary between decrease or increase depending on the ambient light. There are also composition variation systems, especially in high-energy light, but there is no variation system taking into account natural aversion to light sources, since the problem of numbness of the eye was unknown as explained in the present specification.

Document PCT/ES2013/070476 describes a method, device and system for the detection and quantification of variation in eye damage caused by blue and violet light in the visible spectrum, comprising steps of detecting incident radiation on an individual's visual system; calculating the incident radiation inside the range from 380 nm to 500 nm; establishing at least one threshold for incident radiation within said range; detecting if at least one threshold established for this range has been exceeded; warning of the exceedance of at least one threshold; measuring the time of exposure to the incident radiation; and inferring the effect of the incident radiation in the individual's different eye structures, and warning of said effect.

### Description of the invention

The behaviour of the lack of aversion of human ocular system towards a light source is related to the information that reaches the individual's brain. Thus, when an individual is in front of a screen, his/her brain chooses to receive the information and rejects the natural aversion to a light source that has a direct impact on his/her ocular system. This is due to the numbness of the eye for the benefit of the extraction of information that will be most crucial for survival, creating the process a vicious circle where the individual does not rest because he/she does not feel any discomfort or the discomfort he/she feels is not sufficient for the change of activity (for example, because he/she is working)).

This mechanism appears to form part in children and young people in a more intense way, reaching the point that several of the users of the study in ages around 20 years did not feel any discomfort or aversion to the light emitted by the screens.

These levels of aversion are also related to the ambient light. The ocular system behaves differently during the day and at night, so the levels of aversion are different, since the reception capacity of the ocular system is also different.

This problem is very important for development, especially of the retina, since a too prolonged exposure can trigger the death of photoreceptors that, since they form part of the central nervous system, results in irreversible damage. In addition, due to the current levels of exposure to direct light sources as the screens of portable electronic devices, it is expected that ailments such as degeneration macular (AMD) will increase significantly in the future.

It is therefore an object of the invention to detect the level of aversion to a direct-type light source, for example (without limitation) computer screens or those of the portable electronic devices. This detection of the level of aversion will also relate to the level which, according to the age or special condition of his/her ocular system will be considered medically acceptable. This medically acceptable level will be called "healthy aversion" or "acceptable level of aversion".

These objectives are achieved through the method and system of independent claims remaining here incorporated by reference. The dependent claims show not limitative particular embodiments of the present invention and are also incorporated into this specification by reference to the same.

Throughout the description and claims, the word "comprises" and its variations are not intended to exclude other technical features, additives, components or steps. For those skilled in the art, other objects, advantages and characteristics of the invention will emerge in part from the description and in part from the practice of the invention. The following examples and drawings are provided by way of illustration, and are not intended to be limiting of the present invention. Furthermore, the present invention covers all the possible combinations of particular and preferred embodiments herein indicated.

### Brief Description of the Figures

Described very briefly hereinafter are a series of drawings that help to better understand the invention and which are expressly related to an embodiment of said invention that is presented as a non-limiting example thereof.
FIG. 1 - shows a scheme of the portable electronic device that implements the present invention.

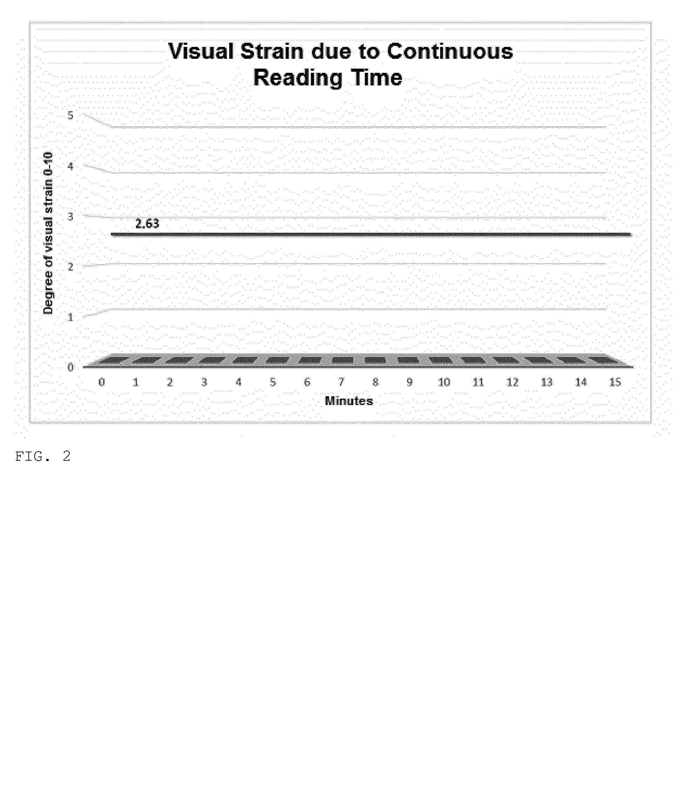

FIG. 2 - shows a graph of the visual strain due to continuous reading time.

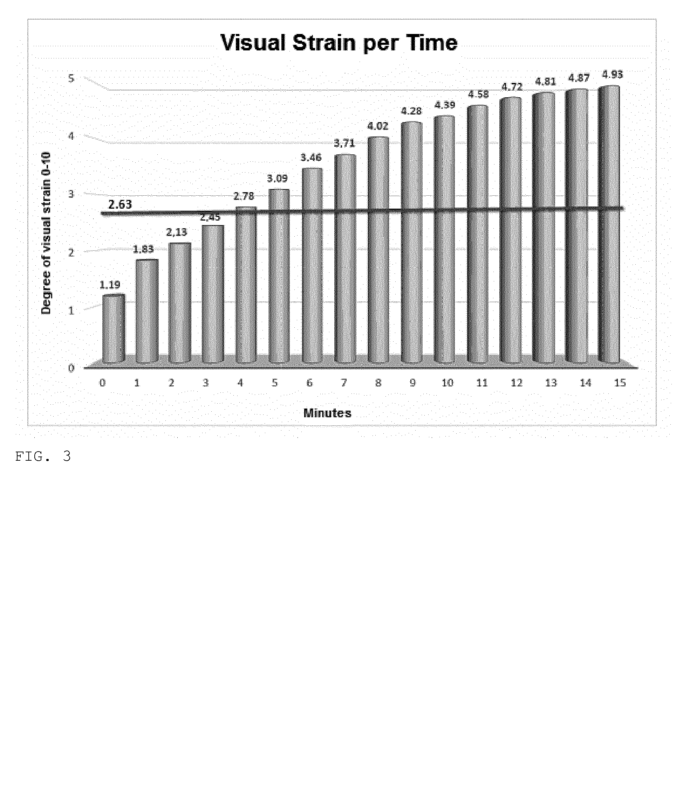

FIG. 3 - shows a graph of the visual strain per minutes.

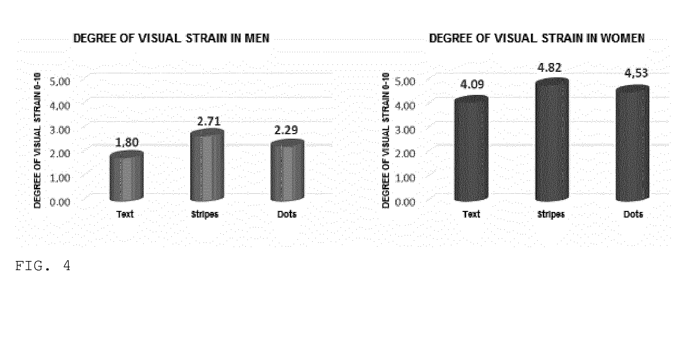

FIG. 4 - shows a graph of the visual strain distributed by sex.

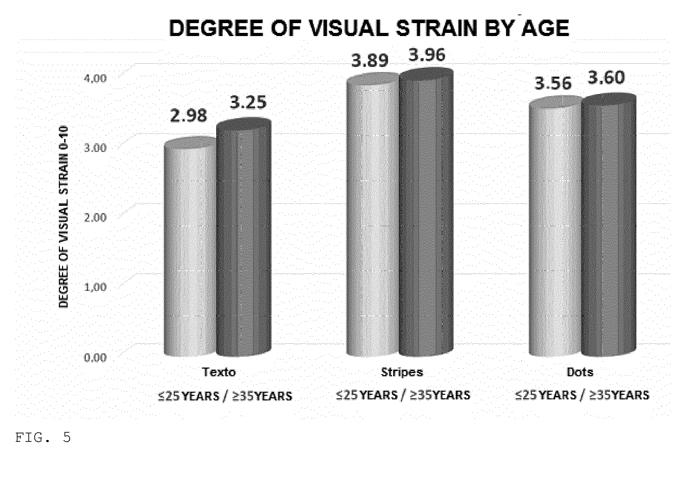

FIG. 5 - shows a graph of the visual strain by age.

### Explanation of a detailed embodiment of the invention

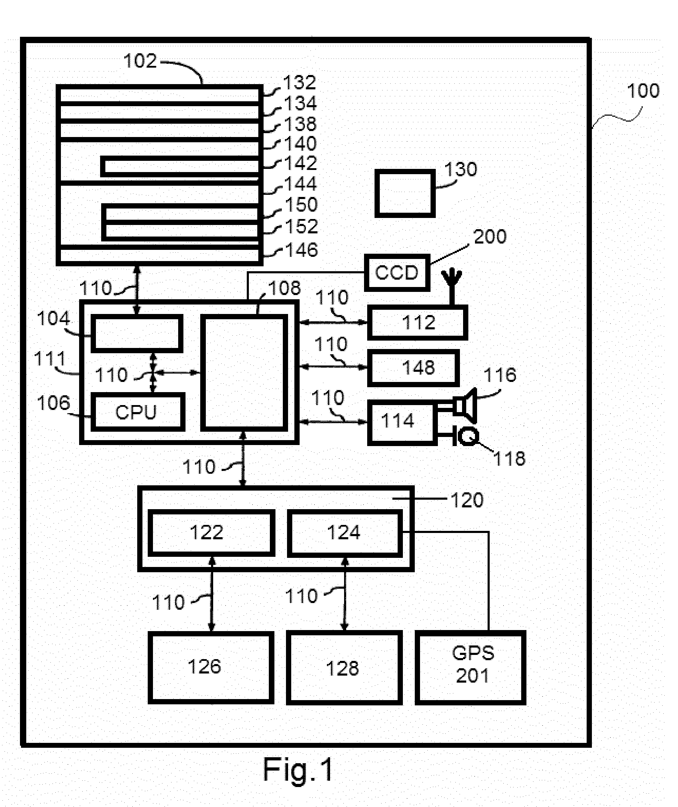

The present invention is implemented in a electronic device or mobile device 100 which may be any selected from computers, tablets and mobile phones, although a preferred architecture for a mobile device is shown in FIG. 1. In general, any programmable electronic device can be configured as a device for the present invention.

FIG. 1 shows an electronic device which according to some embodiments of the invention is a portable electronic device 100. The portable electronic device 100 of the invention includes a memory 102, a memory controller 104, one or more processing units (CPU) 106, a peripherals interface 108, a RF circuitry 112, an audio circuitry 114, a speaker 116, a microphone 118, an input/output (I/O) subsystem 120, a touch screen 126, other input or control devices 128, and an external port 148. These components communicate over the one or more communication buses or signal lines 110. The device 100 can be any portable electronic device, including but not limited to a handheld computer, a tablet, a mobile phone, a media player, a personal digital assistant (PDA), or the like, including a combination of two or more of these items. It should be appreciated that the device 100 is only one example of a portable electronic device 100, and that the device 100 may have more or fewer components than shown, or a different configuration of components. The various components shown in FIG. 1 may be implemented in hardware, software or a combination of both hardware and software, including one or more signal processing and/or application specific integrated circuits. In the same way, the screen 126 is defined as touch-sensitive, although the invention can also be deployed in devices with a standard screen.

The memory 102 may include high speed random access memory and may also include non-volatile memory, such as one or more magnetic disk storage devices, flash memory devices, or other non-volatile solid state memory devices. In some embodiments, the memory 102 may further include storage remotely located from the one or more processors 106, for instance network attached storage accessed via the RF circuitry 112 or external port 148 and a communications network (not shown) such as the Internet, intranet(s), Local Area Networks (LANs), Wide Local Area Networks (WLANs), Storage Area Networks (SANs) and the like, or any suitable combination thereof. Access to the memory 102 by other components of the device 100, such as the CPU 106 and the peripherals interface 108, may be controlled by the memory controller 104.

The peripherals interface 108 couples the input and output peripherals of the device to the CPU 106 and the memory 102. The one or more processors 106 run various software programs and/or sets of instructions stored in the memory 102 to perform various functions for the device 100 and to process data.

In some embodiments, the peripherals interface 108, the CPU 106, and the memory controller 104 may be implemented on a single chip, such as a chip 111. In some other embodiments, they may be implemented on separate chips.

The RF (radio frequency) circuitry 112 receives and sends electromagnetic waves. The RF circuitry 112 converts electrical signals to/from electromagnetic waves and vice versa and communicates with communications networks and other communications devices via the electromagnetic waves. The RF circuitry 112 may include well-known circuitry for performing these functions, including but not limited to an antenna system, an RF transceiver, one or more amplifiers, a tuner, one or more oscillators, a digital signal processor, a CODEC chipset, a subscriber identity module (SIM) card, memory, and so forth. The RF circuitry 112 may communicate with the networks, such as the Internet, also referred to as the World Wide Web (WWW), an Intranet and/or a wireless network, such as a cellular telephone network, a wireless local area network (LAN) and/or a metropolitan area network (MAN), and other devices by wireless communication. The wireless communication may use any of a plurality of communications standards, protocols and technologies, including but not limited to Global System for Mobile Communications (GSM), Enhanced Data GSM Environment (EDGE), wideband code division multiple access (W-CDMA), code division multiple access (CDMA), time division multiple access (TDMA), Bluetooth, Wireless Access

(Wi-Fi) (e.g., IEEE 802.11a, IEEE 802.11b, IEEE 802.11g and/or IEEE 802.11n), voice over IP (VoIP), Wi-MAX, a protocol for email, instant messaging, and/or Short Message Service (SMS), or any other suitable communication protocol, including communication protocols not yet developed as of the filing date of this document.

The audio circuitry 114, the speaker 116, and the microphone 118 provide an audio interface between a user and the device 100. The audio circuitry 114 receives audio data from the peripherals interface 108, converts the audio data to an electrical signal, and transmits the electrical signal to the speaker 116. The speaker converts the electrical signal to human-audible sound waves. The audio circuitry 114 also receives electrical signals converted by the microphone 116 from sound waves. The audio circuitry 114 converts the electrical signal to audio data and transmits the audio data to the peripherals interface 108 for processing. Audio data may be may be retrieved from and/or transmitted to the memory 102 and/or the RF circuitry 112 by the peripherals interface 108. In some embodiments, the audio circuitry 114 also includes a headset jack (not shown). The headset jack provides an interface between the audio circuitry 114 and removable audio input/output peripherals, such as output-only headphones or a headset with both output (headphone for one or both ears) and input (microphone).

The I/O subsystem 120 provides the interface between input/output peripherals on the device 100, such as the touch screen 126 and other input/control devices 128, and the peripherals interface 108. The I/O subsystem 120 includes a touch screen controller 122 and one or more input controllers 124 for other input or control devices. The one or more input controllers 124 receive/send electrical signals from/to other input or control devices 128. The other input/control devices 128 may include physical buttons (e.g., push buttons, rocker buttons, etc.), dials, slider switches, and/or geographical location means 201, such as GPS or similar.

In this practical embodiment, the screen 126 is touch-sensitive and provides both an output interface and an input interface between the device and a user. The touch screen controller 122 receives/sends electrical signals from/to the touch screen 126. The touch screen 126 displays visual output to the user. The visual output may include text, graphics, video, and any combination thereof. Some or all of the visual output may correspond to user-interface objects, further details of which are described below.

In this practical embodiment, the screen 126 of the invention is configured as touch sensitive, however, any screen, whether touch sensitive or not, would be valid for the present invention.

The touch screen 126 also accepts input from the user based on haptic and/or tactile contact. The touch screen 126 forms a touch-sensitive surface that accepts user input. The touch screen 126 and the touch screen controller 122 (along with any associated modules and/or sets of instructions in the memory 102) detects contact (and any movement or break of the contact) on the touch screen 126 and converts the detected contact into interaction with user-interface objects, such as one or more soft keys, that are displayed on the touch screen. In an exemplary embodiment, a point of contact between the touch screen 126 and the user corresponds to one or more digits of the user. The touch screen 126 may use LCD (liquid crystal display) technology, or LPD (light emitting polymer display) technology, although other display technologies may be used in other embodiments. The touch screen 126 and touch screen controller 122 may detect contact and any movement or break thereof using any of a plurality of touch sensitivity technologies, including but not limited to capacitive, resistive, infrared, and surface acoustic wave technologies, as well as other proximity sensor arrays or other elements for determining one or more points of contact with the touch screen 126.

The device 100 also includes a power system 130 for powering the various components. The power system 130 may include a power management system, one or more power sources (e.g., battery, alternating current (AC)), a recharging system, a power failure detection circuit, a power converter or inverter, a power status indicator (e.g., a light-emitting diode (LED)) and any other components associated with the generation, management and distribution of power in portable devices.

In some embodiments, the software components include an operating system 132, a communication module (or set of instructions) 134, a contact/motion module (or set of instructions) 138, a graphics module (or set of instructions) 140, a user interface state module (or set of instructions) 144, and one or more applications (or set of instructions) 146.

The operating system 132 (e.g., Darwin, RTXC, LINUX, UNIX, OS X, WINDOWS, or an embedded operating system) includes various software components and/or drivers for controlling and managing general system tasks (e.g., memory management, storage device control, power management, etc.) and facilitates communication between various hardware and software components.

The communication module 134 facilitates communication with other devices over one or more external ports 148 and also includes various software components for handling data received by the RF circuitry 112 and/or the external port 148. The external port 148 (e.g., Universal Serial Bus (USB), FIREWIRE, etc.) is adapted for coupling directly to other devices or indirectly over a network (e.g., the Internet, wireless LAN, etc.).

The contact/motion module 138 detects contact with the touch screen 126, in conjunction with the touch screen controller 122. The contact/motion module 138 includes various software components for performing various operations related to detection of contact with the touch screen 126, such as determining if contact has occurred, determining if there is movement of the contact and tracking the movement across the touch screen, and determining if the contact has been broken (i.e., if the contact has ceased). Determining movement of the point of contact may include determining speed (magnitude), velocity (magnitude and direction), and/or an acceleration (including magnitude and/or direction) of the point of contact. In some embodiments, the contact/motion module 126 and the touch screen controller 122 also detects contact on the touchpad.

The graphics module 140 includes various known software components for rendering and displaying graphics on the touch screen 126. Note that the term "graphics" includes any object that can be displayed to a user, including but not limited to, text, web pages, icons (such as user-interface objects including soft keys), digital images, videos, animations and the like.

In some embodiments, the graphics module 140 includes an optical intensity module 142. The optical intensity module 142 controls the optical intensity of graphical objects, such as user-interface objects, displayed on the touch screen 126. Controlling the optical intensity may include increasing or decreasing the optical intensity of a graphical object. In some embodiments, the increase or decrease may follow predefined functions.

This will require a system for detecting the light emitted according to the present invention. There are several formulas to do it, the first is to know the emission using the system data and the time of exposure, although it is true that there is a temporal evolution that decreases the amount of light that the screen emits so it is necessary to check the amount of light that the user is receiving, a formula is to use a camera 200 in order to see the reflection produced by the image of eyes on the screen and thereafter relate it to a healthy reflection pattern for the ocular system.

Another method is to manage emissions and composition based on a maximum standard quantities of emission taking into account either the average behaviour of the eye or the particular behaviour of a given age or physiognomy of the person, for example, someone with blue eyes will need more aversion than someone who has cataracts who need less aversion, i.e. the entire population can be included in the threshold and arbitrate a behaviour of the screen so that it has into account the aversion in a manner generic, particular to each individual or group, for each segment of the population that share a number of common features among them.

For this purpose, devices will reduce its emission to an amount which will vary related to factors such as ambient lighting and the composition of the emission, and that will take into account that discovered factor of aversion to the source.

There also are discomfort reactions to different colours, some in particular have the ability to generate or producing headaches since certain wavelengths are associated with brain reactions that trigger rejection in people.

Visual activity of the individual can also be taken into account, and an activity like reading that affects more to the macula will have more risk that seeing a video, that affects less to central region and can be less risky for the retina.

Also the amount of time and the decrease in the emission related to the aversion.

A procedure is also defined to arouse the individual's aversion including a series of steps of exposure to light and contents to activate in one greater way the aversion or to bring to the conscious area the perception of this difference between the natural aversion and that induced by the information.

This device or software application consists of a test of aversion where the person will responds to the feelings of discomfort offered by the screen and so this can be configured in a way more consistent with an aversion closer to the natural aversion.

By way of example the application is described, the device has a screen with an image and analyzes the person's response to the question of the level of discomfort that this picture produces in the person, system may increase the intensity or change the composition or both, the change of composition will be made by replacing a range of colours or one colour by other one, so that in reply to the questions about the comfort or discomfort of the image for the person's eyes, a series of preferences and conclusions are created to activate a visual behaviour pattern that is capable of learning in the course of the different exposures which an individual may have.

Through carried out experiments, it is concluded that part of individuals react with defensive moves due to the excess light that are detectable by the device through a camera with visual access to the eyes and surrounding areas of the eye.

The user interface state module 144 controls the user interface state of the device 100. The user interface state module 144 may include a lock module 150 and an unlock module 152. The lock module detects satisfaction of any of one or more conditions to transition the device 100 to a user-interface lock status and to transition the device 100 to the lock status. The unlock module detects satisfaction of any of one or more conditions to transition the device to a user-interface unlock status and to transition the device 100 to the unlock status.

The one or more applications 130 can include any applications installed on the device 100, including but not limited to, a browser, address book, contact list, email, instant messaging, word processing, keyboard emulation, widgets, JAVA-enabled applications, encryption, digital rights management, voice recognition, voice replication, location determination capability (such as that provided by the global positioning system (GPS)), a music player (which plays back recorded music stored in one or more files, such as MP3 or AAC files), etc.

In some embodiments, the device 100 may include one or more optional optical sensors (not shown), such as CMOS or CCD image sensors, for use in imaging applications.

This camera 200 can also measure by comparing the gestures of the user when he/she is reading, a gesture of the eye will indicate a defensive physiological response, however, a relaxed eye will lead to a more natural physiological acceptance of the light intensity, this comparison can be made with own or external patterns, the own patters can be created with exposure to an amount of light estimated as high and compare it with an amount of light estimated as of low energy or healthier.

This camera can be on the device itself or, for example, in glasses that allow detecting these behaviours with greater clarity, and in turn sending the data to the computer or the screen so that this changes its composition, intensity, or behaviour.

However, the indicated hardware structure is one of the possible and it must be taken into account that the device 100 may include other elements for capturing images such as a camera, scanner, laser plotter or the combination of any of these types of devices, which may provide the mobile device the display of the actual environment in video format, sequence of images, vector format or any combination of the above formats.

Similarly, the device 100 may include geographic location devices based on networks of GPS positioning satellites, devices of geographical location aid, based on GPS satellite networks, and IP location of Internet networks -AGPS-, geographical location devices based on the triangulation of radio signals provided by WIFI antennas and Bluetooth® devices (ISSP), the combination of any of these mentioned devices or any type of device which allows providing the mobile device numerical data of its geographical location.

The device 100 may include any type of element capable of displaying images in real time with a minimum of 24 FPS (Frames Per Second) such as TFT, TFT-LED, TFT-OLED, TFT-Retina displays, the combination of any of the above as well as new generation Holo-TFT and transparent displays, and Micro-Projectors, or any graphic display device that can provide the mobile device 100 a way of displaying visual content for the user.

The device 100 includes a processor or set of processors that by themselves or in combination with graphics processors such as GPU (Graphics Processing Unit) or APU (Accelerated Processing Unit) can provide the mobile device 100 the ability to display, in real time, vector graphics and conform polygons textured with these, through libraries for vector representation (sets of standardized procedures of graphic representation for different platforms) such as OpenGL, DirectX or any type of libraries intended for this purpose.

Thus, the system of the invention identifies a level of aversion for the individual and relates it to or not with the abovementioned healthy level of aversion to emit the user an amount and intensity of light. In this sense the system can be configured to only reduce the intensity, keeping intact the colours, the reason lies in that there are professions like graphic designers, doctors who have to observe the coloration of a tumour in a vision system of the human body, or others where a slight difference of tone can be crucial for the outcome of their work and who need a system for reducing and managing colour that reduces their emissions by keeping the fidelity thereof. Therefore, the system can be configured to only vary the intensity.

### Aversion Assay

Preliminary studies carried out at Complutense University of Madrid (College of Optics and Optometry), show new findings that would explain the lack of alarm against the high-energy light emitted by the screens of electronic devices.

The defence systems against the light are "disabled or numb" when interacting with the content or information on the screens. Our eyes are not prepared to defend themselves against the light. We do not realize the harmful effects of excess light, even we are not aware of some symptoms and signs while we are carrying out activities in front of the screens.

The experiment consisted in extensive tests carried out on a group of 26 men and women aged between 21 and 50 years; the symptoms of visual strain were analysed by segmenting the sample in different age groups.

The methodology used was as follows:
The study lasted for 1 hour and 20 minutes (80 minutes). The individual was located at 40 cm from a 9-inch tablet. The room lighting was under photopic condition (100 cd/m²). After 5 minutes of adaptation in the light of the room, the first phase began which was that the individual read for 15 minutes a coherent text obtained from a novel; at the end of testing, he/she was asked about his/her state of visual strain and asked data relating to the read contents, to know his/her degree of concentration and understanding of the text. In the second part of the experiment, and randomly, the same device displayed coherent text, stripes or spots, and unlike the first phase of the test, he/she was asked about the degree of visual strain every minute, scoring the results in a progressive manner. Between each reading, he/she had been left to rest for 5 minutes with eyes closed. Finally, a questionnaire was made with questions about symptoms and signs, attached in the corresponding file.

The visual strain was manifested in all ages, being an interesting finding to know that it is significantly increased in persons over the age of 35, after the use of device screens during about two hours of work/study. 92 % of respondents indicated that they felt any symptom of fatigue in their eyes in a frequent or constant manner, while 42 % reported from 1 to 5 symptoms, 35 % from 6 to 10 symptoms, and 15 %, more than 10 symptoms.

As relevant data, it should be noted that women report double eye sensitivity, in high-energy light of the screens of electronic devices, than men (4.24/2.13). 17% of young people under 25 years does not report any kind of visual strain, i.e., its natural defence mechanism does not warn him/her of eye risk, possibly as a result of seduction that the content has.

On the other hand, it was detected, as expected, that the visual strain increases according to the time of observation of the screen, increasing discomfort up to four times more from the beginning until the end of the test (scale of 0 to 10/15 minutes of reading on a screen). These preliminary tests indicate that they must take into account the hidden risks of routine use of device screens that may be a threat to our eyes. Therefore, we want to alert the population about the existence of hidden risks due to lack of activation of our physiological alert mechanisms against possible visual damages. It seems that two psychophysiological processes come into conflict; on the one hand, the need to assimilate the content of the screen, and on the other hand, the essential protection of abuse of our eyes.

The study of the UCM contributes to better understand the behaviour of the eye with respect to the receipt of information and content on the screens of devices. This work makes a new contribution to the analysis of a habit so common and global as it is the continued use of electronic devices, by emphasising that defence mechanisms are "disabled or numb" when reading contents and information on the screens.

**Table 1. Twelve signs and symptoms associated with the use of electronic devices**

| | |
|---|---|
| Visual strain | 62 % |
| Photophobia in screen | 38 % |
| Ocular heaviness | 35 % |
| Difficulty of focusing | 35 % |
| Photophobia in high contrast situation | 31 % |
| Eye redness | 31 % |
| Blur in near vision | 31 % |
| Eye pain | 27 % |
| Eye itching | 23 % |
| Dryness of eye | 23 % |
| Headache | 15 % |
| Tearing | 12 % |

## Claims

1. Computer implemented method comprising the steps of:
a) detecting the level of light emission that a light source emits into a user's ocular system;
b) measuring the user's level of aversion to the amount of light received in his/her ocular system through analysis of the user's eye reaction; and
c) comparing the level of aversion to the amount of light received by his/her ocular system measured in step (b) to the level of aversion acceptable for that user and stored in a memory (102) of an electronic device (100).

2. The method of claim 1 further comprising a step of reducing the light emission of a light source to equal the acceptable level of aversion with the user's measured level of aversion.

3. A portable electronic device (100), comprising:
a screen (126);
one or more processors (106);
a memory (102);
a camera (200); and
one or more programs (136 to 146) wherein the program(s) (132 to 146) are stored in the memory (102) and configured to be executed by the processor(s) (106); **characterized in that** the programs (132 to 146) include instructions for:
a) detecting the level of light emission that a light source emits into a user's ocular system;
b) measuring the user's level of aversion to the amount of light received in his/her ocular system through analysis of his/her eye reaction through at least one camera (200);
c) comparing the level of aversion to the amount of light received by his/her ocular system measured in step (b) to the level of aversion acceptable for that user and stored in a memory (102) of an electronic device (100).

4. The device (100) according to claim 3 comprising programmes including instructions to reduce the light emission of a light source to equal the acceptable level of aversion with the user's measured level of aversion.

5. The device (100) according to any of claims 3 or 4 that is portable.

6. A software product with instructions configured for execution by one or more processors that, when executing by an electronic device (100) according to any of claims 3 to 5, causes said device (5) to carry out the method according to any of claims 1 to 2.
